# EUROPÄISCHE PATENTSCHRIFT

(11) **EP 1 517 985 B1**
(45) Veröffentlichungstag und Bekanntmachung des Hinweises auf die Patenterteilung: **23.05.2007**
(21) Anmeldenummer: 03704654.7
(22) Anmeldetag: 19.02.2003
(51) Int. Cl.: C12M 1/16, C12M 1/40

(54) **KULTIVIERUNGSVERFAHREN FÜR MIKROORGANISMEN UND BIOREAKTOR**
CULTIVATION METHOD FOR MICRO-ORGANISMS AND BIOREACTOR
PROCEDE DE CULTURE DE MICRO-ORGANISMES ET BIOREACTEUR

(30) Priorität: 19.02.2002 DE 10206772
(43) Veröffentlichungstag der Anmeldung: 30.03.2005
(73) Patentinhaber: Höfer Bioreact GmbH, 53115 Bonn (DE)
(72) Erfinder: HÖLKER, Udo, 53115 Bonn (DE)
(74) Vertreter: Helbing, Jörg
(86) Internationale Anmeldenummer: PCT/EP2003/001663
(87) Internationale Veröffentlichungsnummer: WO 2003/070873

(56) Entgegenhaltungen:
- EP-A- 0 442 110
- WO-A-01/02307
- WO-A-92/18237
- DE-A- 3 227 678
- DE-C- 4 308 832
- DE-C- 19 511 159
- DE-C- 19 609 053
- US-A- 4 351 729
- US-A- 4 746 615
- US-A- 5 510 262
- SORIAL G A ET AL.: "Evaluation of trickle-bed air biofilter performance for styrene removal" WATER RESEARCH, Bd. 32, Nr. 5, 1. Mai 1998 (1998-05-01), Seiten 1593-1603, XP004122127 ISSN: 0043-1354
- PATENT ABSTRACTS OF JAPAN vol. 012, no. 165 (C-496), 18. Mai 1988 (1988-05-18) -& JP 62 277195 A (SANKI ENG CO LTD), 2. Dezember 1987 (1987-12-02)
- PATENT ABSTRACTS OF JAPAN vol. 016, no. 029 (C-0904), 24. Januar 1992 (1992-01-24) & JP 03 242222 A (KUBOTA CORP), 29. Oktober 1991 (1991-10-29)
- PATENT ABSTRACTS OF JAPAN vol. 010, no. 103 (C-340), 18. April 1986 (1986-04-18) -& JP 60 232295 A (KURITA KOGYO KK), 18. November 1985 (1985-11-18)
- PATENT ABSTRACTS OF JAPAN vol. 014, no. 458 (C-0766), 3. Oktober 1990 (1990-10-03) -& JP 02 184398 A (NKK CORP), 18. Juli 1990 (1990-07-18)
- PATENT ABSTRACTS OF JAPAN vol. 2000, no. 24, 11. Mai 2001 (2001-05-11) -& JP 2001 205283 A (SANYO ELECTRIC CO LTD), 31. Juli 2001 (2001-07-31)

## Beschreibung

Die Erfindung betrifft ein Kultivierungsverfahren für Mikroorganismem sowie einen hierfür geeigneten Bioreaktor. In diesem Bioreaktor wird das Prinzip des Rieselbettverfahrens mit dem des Wirbelbettverfahrens kombiniert und ein völlig neues Kultivierungsprinzip mit Hilfe dieses Bioreaktortyps zur Verfügung gestellt.

### Hintergrund der Erfindung

Die Grundprinzipien und Applikationen des Rieselfilmverfahrens, z.B. biologische Abwasserreinigung, biologische Abluftreinigung und Anwendung zur Hydrogenierungen etc. und die des Wirbelbettverfahrens (gelegentlich auch als Fließbettverfahren bezeichnet), z. B. anaerobe Abwasserbehandlung, Nitrifikation und Denitrifikation von Trink- und Abwasser etc. sind bekannt.

Die Hauptschwierigkeit beim Betrieb von Rieselfilmreaktoren und den entsprechenden Verfahren wie z.B. der genannten Abluftreinigung liegen in der Tatsache begründet, dass die durch die Verstoffwechslung der angebotenen Substrate entstehende Biomasse zum Zuwachsen und letztendlich zum Verstopfen des Reaktors führt, da die freien Zwischenräume der Füllkörperschüttung bzw. des festen Substrats durch die Biomasse zugesetzt werden. Dadurch steigt der Druckverlust über dem Festbett an und die Abbauleistung bzw. Produktionsleistung wird durch Kanalbildung und Todzonen erheblich vermindert. Dies ist insbesondere bei Rieselfilmreaktoren mit hohen Raum-Zeitausbeuten der Fall, die entsprechend sehr schnell verblocken können.
Es gibt verschiedene patentierte und aus der Literatur bekannte Systeme, die mittels verschiedener Verfahrensvarianten Lösungen für diese Problematik aufzeigen.

So wird in den VDI-Berichten 1104 (1994) die Verzögerung des Aufwachsens der Biomasse durch z.B. N-Limitierung beschrieben. Der Nachteil ist, dass die Verblockung nur hinausgezögert und nicht grundsätzlich verhindert wird und zudem die entsprechenden Abbauleistungen stark reduziert sind. In DE 3227678 wird ein Verfahren beschrieben, bei dem über den gesamten Reaktionsverlauf nicht nur Rieselwasser zugeführt wird, sondern soviel Wasser, dass gleichzeitig auch überschüssiger Bewuchs mitgerissen wird, indem das Wasser durch die Zwischenräume des Trägerkörpers herabrieselt. Zusätzlich wird der Reaktorkörper zeitweise geflutet und mit dem Flutungswasser von unten nach oben durchströmt. Dabei werden die dort beschriebenen speziellen Microcarrier aufgeschwemmt und gegeneinander verwirbelt, wobei die überschüssige Biomasse durch Schwerkräfte abgelöst und gegebenenfalls mit den Microcarriern zusammen ausgetragen wird. Dieses Verfahren ist aber nicht geeignet die Füllkörperschüttung erfolgreich zu reinigen, da die Scherwirkung, die aus dem Aufschwemmen des Füllkörpers alleine resultiert, zu gering ist, um eine Verblockung nachhaltig zu verhindern. Ähnliches gilt für die in DE 3938507 und DE 3204597 dargestellten Verfahren, die eine Auflockerung mittels Rührereinbau beschreiben, was allerdings für längere Standzeiten ebenfalls unzureichend ist.

DE 19608805 beschreibt ein Verfahren, welches eine Auflockerung mittels Schneckenrührung und Fluten beschreibt, wobei die Rührzeit zwischen 5 bis maximal 60 min beträgt und die Drehzahl zwischen 3 bis maximal 30 U/min liegt. Gleichzeitig wird ein Wasserstrom von mindestens 0.05 m³ pro m² und Stunde bis maximal 5m³ pro m² und Stunde zur Flutung des Reaktorraumes eingesetzt.

In DE 19609053 wird ein Verfahren benannt, bei dem das Festbett zunächst geflutet und später die Festbettschüttlung von unten nach oben mit Flutungswasser durchströmt wird, wobei gleichzeitig Gas parallel mit steigender Geschwindigkeit durch das Festbett hindurchgeleitet wird und ferner Druckgaspulse gegeben werden. Hierdurch kommt es zu Turbulenzen im Festbett, wodurch die überschüssige Biomasse durch Scherkräfte abgelöst und über den Flüssigkeitsstrom ausgetragen wird. Ein Wirbelbett wird nicht genannt.

Sorial, G.A. et al., Water Res. 32:1593-1603 (1998), beschreiben einen "trickle-bed air biofilter", also einen Rieselbett-Luftfilter zur Entfernung von Styrol aus einem Abgasstrom. Als Füllkörper dienen Celite Kugeln, welche als Bindungselemente zur Verankerung der Biomasse dienen. Der Filter wird durch einen "Backwash"-Vorgang von überschüssiger Biomasse gereinigt.
DE 195 11 159 betrifft ein Verfahren zur Behandlung von Abwasser, wobei sich in dem Behandlungsbecken Käfige befinden, welche teilweise mit kugelförmigen Trägern gefüllt sind. Die Träger schweben im Abwasser und bieten Mikroorganismen eine Aufwuchsfläche. Die Käfige werden in Abhängigkeit von der Abwasserbelastung als Rieselfilm- oder Wirbelschichtreaktoren betrieben.

Dauer und Stärke der in den genannten Verfahren erzeugten Scherkräfte zur Auflockerung des Festbetts mögen für spezielle Anwendungen (Abgasreinigung vornehmlich mit Hilfe von Bakterien) ausreichen, tun dies jedoch nicht allgemein. Insbesondere kommt es bei der biotechnologischen Nutzung mancher filamentöser Pilze in Rieselbettverfahren nicht nur zu einem Bewachsen der Mikropartikel sondern auch - durch die Bildung von Hyphen - zu einer Vernetzung der Partikel untereinander, wodurch der Grad der Verblockung wesentlich erhöht ist.

### Kurzbeschreibung der Erfindung

Aufgabe der Erfindung ist es, einen Bioreaktor und ein Kultivierungsverfahren von Mikroorganismen zur Verfügung zu stellen, das die Nachteile des oben genannten Stands der Technik nicht aufweist. Insbesondere sollte der Bioreaktor/das Kultivierungsverfahren nicht wie bekannte Rieselfilmreaktoren zum Verstopfen neigen und insbesondere für die Anwendungen
a) Produktion von Proteinen,
b) Produktion von Sekundärmetaboliten,
c) Behandlung von pflanzlichem Material (Holz, Faserstoffe, Einjahrespflanzen),
d) Einsatz als Biokatalysatorsystem bei der chemischen Synthese,
e) Einsatz in der Schadstoffentfernung aus Abwasser und
f) Abbau von Xenobiotika,
verbunden mit der gleichzeitigen Möglichkeit der Produktanalyse, Produktextraktion und Produktvorreinigung (a-d), geeignet sein.

Völlig überraschend wurde gefunden, dass bei einer Kombination des klassischen Rieselbettverfahrensprinzips mit dem klassischen Wirbelbettverfahrensprinzip die Nachteile des Standes der Technik nicht mehr auftreten. Bei dem neuen Verfahrensprinzip wird während der Wirbelbettverfahrensweise das Wirbelbett extern begast und dabei optional zu Beginn oder während der Wirbelbettverfahrensweise zusätzlich Druckgaspulse zur Auflockerung des Festbetts gegeben. Es verhindert vor allem eine Verblockung der entsprechenden Kulturen bei Batch-, Fedbatch und vor allem kontinuierlicher Kulturführung mit langen Standzeiten (wie aus ökonomischen Gründen meistens gefordert).

Zusätzlich werden als Vorteile die zwingend erforderliche Separierung/Aufteilung der Füllkörper von Biofilmen bzw. bei Pilzbewuchs die Separierung/ Aufteilung der Mattenstruktur erreicht, was durch die erfindungsgemäß kombinierte Wirbelbettverfahrweise mit Hilfe des neuen Reaktortyps zu einem wesentlich verbesserten (Neu-)Anwachsen der vermehrten "Inokulationskeimzellen" bei der nachfolgenden Rieselfilmreaktor-Fahrweise führt, sodass also nicht nur die Verblockung aufgehoben wird, sondern auch die Produktivität und/oder die Abbauraten stark erhöht werden können.

Die Erfindung betrifft somit
(1) ein Kultivierungsverfahren für Mikroorganismen, umfassend eine Kombination aus Verfahrensschritten, die für einen Rieselbettreaktor charakteristisch sind, mit solchen, die für einen Wirbelbettreaktor charakteristisch sind, wobei
   (a) Mikroorganismenkulturen auf einem Festbett unter Rieselbettreaktor-Verfahrensführung kultiviert werden, wobei das Reaktionsmedium durch Rieseldüsen aufgebracht wird, die Belüftung von unterhalb und/oder innerhalb des Festbetts erfolgt und die durch die Festbettschicht ablaufende Lösung, welche die Reaktions-/Abbauprodukte der Mikroorganismenkulturen enthält, entweder solange, bis die gewünschte Produktivität, das gewünschte Produkt bzw. das gewünschte Abbauprodukt oder der gewünschte Abbaugrad erreicht sind, zurückgeführt wird oder die abgelaufene Lösung unmittelbar zur weiteren Auftrennung weiterbehandelt wird und
   (b) bei Überschreiten eines bestimmten Druckverlustes über die Befüllung des Festbetts durch Einleiten von Wasser oder Nährlösung und durch Einleiten von Luft die Kulturführung für einen gewissen Zeitraum von der Rieselbettreaktor-Verfahrensführung in eine Wirbelbettreaktor-Verfahrensführung umgeschaltet wird, um gegebenenfalls einem Verstopfen des Festbettes zu begegnen;
(2) einen Bioreaktor zur Kultivierung von Mikroorganismen, insbesondere zur Durchführung des unter (1) beschriebenen Verfahrens, mit
   einem Reaktionsbehälter (10),
   einer in dem Reaktionsbehälter (10) angeordneten Festbettauflage (14) zum Anordnen eines Festbettes (12) auf der Festbettauflage (14),
   oberhalb der Festbettauflage (14) angeordneten Rieseldüsen (18) zur Berieselung des Festbettes (12) mit Nährstofflösung, so dass der Bioreaktor im Rieselbettverfahren betrieben werden kann,
   einer Belüftungseinrichtung (30, 32, 34, 36) zur Belüftung des Festbettes (12) bzw. des Innenraums des Reaktors (10), so dass der Bioreaktor im Wirbelbettverfahren betrieben werden kann, und
   einer Messwerterfassung (58) zur Erfassung des Drucks außerhalb des oder im Festbett (12); und
(3) einen wie in (2) beschriebenen Bioreaktor mit
   einem Reaktionsbehälter (10),
   einer in dem Reaktionsbehälter (10) angeordneten Festbettauflage (14) zum Anordnen eines Festbettes (12) auf der Festbettauflage (14),
   oberhalb der Festbettauflage (14) angeordneten Rieseldüsen (18) zur Berieselung des Festbettes (12) mit Nährstofflösung oder dgl., so dass der Bioreaktor im Rieselbettverfahren betrieben werden kann und
   einer mit dem Reaktionsbehälter (10) verbundenen Druckerzeugungseinrichtung (80) zum Hindurchpressen von Reaktionsmedium (56) durch die Festbettauflage (14) und insbesondere durch die unterhalb der Festbettauflage (14) angebrachten Filter (64, 66).

Das erfindungsgemäße Verfahren (1) ist
a) zur Produktion von Proteinen,
b) zur Produktion von Sekundärmetaboliten,
c) zur Behandlung von pflanzlichem Material (Holz, Faserstoffe, Einjahrespflanzen),
d) zum Einsatz als Biokatalysatorsystem bei der chemischen Synthese,
e) zum Einsatz in der Schadstoffentfernung aus Abwasser und Luft und
f) zum Abbau von Xenobiotika
verbunden mit der gleichzeitigen Möglichkeit der Produktanalyse, Produktextraktion und Produktvorreinigung (a-d) aus der Flüssigkeit geeignet.

Der erfindungsgemäße Bioreaktor (2) zur Aufzucht von Mikroorgansismenkulturen, der insbesondere zur Durchführung des vorstehend beschriebenen erfindungsgemäßen Verfahrens geeignet ist, weist einen Reaktionsbehälter auf. Innerhalb des Reaktionsbehälters ist auf einer Festbettauflage, wie einem Sieb oder einem Gitter, ein Festbett angeordnet. Oberhalb des Festbettes sind entsprechend einem Rieselbett-Reaktor zur Berieselung des Festbettes mit Nährstofflösung Rieseldüsen angeordnet. Die Rieseldüsen sind vorzugsweise regelmäßig über die gesamte Fläche des Festbettes verteilt angeordnet. Erfindungsgemäß weist der Bioreaktor ferner eine Belüftungseinrichtung auf, die oberhalb, innerhalb und/ oder unterhalb des Festbettes angeordnet sein kann. Durch die Belüftungseinrichtung ist ein Zuführen von Luft oder anderen Gasen möglich, um das Fließbett zu belüften, so dass der Reaktor im Wirbelbettverfahren betrieben werden kann. Durch das Zuführen von Luft oder anderen Gasen, beispielsweise in regelmäßigen Abständen oder in Abhängigkeit eines Druckverlustes über dem Festbett, kann ein Verstopfen oder Verblocken des Festbettes und die hierdurch hervorgerufenen Beeinträchtigungen der Reaktionsabläufe vermieden werden.

Bei dem erfindungsgemäßen Bioreaktor (3) ist eine Druckerzeugungseinrichtung mit dem Reaktionsbehälter verbunden. Durch die Druckerzeugungseinrichtung kann innerhalb des Reaktionsbehälters Druck erzeugt werden, durch den das im Festbett entstehende Reaktionsmedium durch das Rückhalteelement und ggf. weitere, innerhalb des Reaktionsbehälters angeordnete Siebe oder Gitter hindurchgedrückt werden kann. Durch dieses vorzugsweise kurzzeitige und/ oder stoßweise Erhöhen des Drucks ist ein Verstopfen bzw. Verblocken des Festbettes sowie ggf. vorhandener Filter etc. vermieden bzw. kann gelöst werden.

Die beiden vorstehend beschriebenen Ausführungsformen weisen vorzugsweise eine oberhalb der Festbettauflage, insbesondere oberhalb des Festbettes, und einer Rückhaltevorrichtung (z. B. einen Sieb oder dergleichen) für die Wirbelbettreaktorführung angeordnete Entnahmeeinrichtung auf. Hierdurch ist eine Mediumabfuhr möglich. Das abgeführte Medium kann im Kreislauf gepumpt werden. Dies ist insbesondere beim Fluten des Reaktors vorteilhaft, wobei durch das Fluten des Reaktors die Reinigung des Rückhalteelements sowie ggf. vorgesehener Filter und Siebe sowie das Verhindern des Verstopfens des Festbettes unterstützt werden kann.

Bei einer besonders bevorzugten Weiterbildung der vorstehend beschriebenen Erfindungen ist unterhalb der Festbettauflage mindestens ein Filterelement angeordnet. Durch das Filterelement kann das Reaktionsmedium noch innerhalb des Reaktors gefiltert bzw. gereinigt werden. Vorzugsweise sind hierbei in Flussrichtung des Reaktionsmediums mehrere Filterelemente hintereinander angeordnet, wobei sich deren Maschenweite bzw. Porengröße in Flussrichtung, d.h. in Richtung eines Auslasses des Bioreaktors verringert.

Vorzugsweise ist den Filterelementen eine Spüleinrichtung zur Reinigung der Filterelemente zugeordnet. Insbesondere weist dieses Filterelement eine gesonderte Spüleinrichtung auf. Mit Hilfe der Spüleinrichtung kann eine Spülflüssigkeit über das Filterelement bewegt werden. Dies kann beispielsweise durch Düsen erfolgen, die eine Spülflüssigkeit auf die Filterelemente spritzt. Hierdurch ist die Gefahr des Verstopfens der Filterelemente verringert. Besonders bevorzugt ist es, die Filterelemente geneigt anzuordnen, so dass eine Spülflüssigkeit über die Filter fließen und dabei die Verunreinigungen herausschwemmen kann. Hierzu kann je Filterelement ein zusätzlicher Auslass vorgesehen sein, um Spülflüssigkeit zusammen mit herausgewaschenen Verunreinigungen abführen zu können. Durch die Neigung der Filterelemente ist ferner die Gefahr des Verstopfens auch während des Reaktionsprozesses verringert, da auch das Reaktionsmedium zum Teil über die Filterelemente fließt und hierbei Verunreinigungen abtransportiert.

### Kurzbeschreibung der Figuren

Nachfolgend wird die Erfindung anhand bevorzugter Ausführungsformen unter Bezugnahme auf die anliegenden Zeichnungen näher erläutert. Es zeigen:
- Fig. 1: eine vereinfachte Prinzipskizze des erfindungsgemäßen Bioreaktors mit Belüftungseinrichtung,
- Fig. 2: eine schematische Draufsicht des in Fig. 1 dargestellten Bioreaktors,
- Fig. 3: eine detaillierte Prinzipskizze des in Fig. 1 dargestellten Bioreaktors,
- Fig. 4: eine Prinzipskizze des in den Fig. 1 - 3 dargestellten Bioreaktors mit zusätzlichen Filterelementen und
- Fig. 5: eine schematische Schnittansicht in Längsrichtung des in Fig. 4 dargestellten Bioreaktors.

### Detaillierte Beschreibung der Erfindung

Bei dem erfindungsgemäßen Kultivierungsverfahren (1) der Erfindung sind die Mikroorganismenkulturen vorzugsweise ausgewählt aus Bakterien, Hefen, Pilzen, tierischen oder pflanzlichen Zellen (einschließlich rekombinanter und immortalisierter Zellen), die immobilisiert sein können, und immobilisiertem pflanzlichen und tierischen Gewebe. Besonders bevorzugt sind filamentöse Pilze.

Weiterhin bevorzugt ist, dass bei der Wirbelbettverfahrensweise das Medium im Kreis gepumpt wird. Durch die Wirbelbettverfahrensweise wird ein Überlaufen von bewachsenem Füllkörper oder bewachsenen Festbettbestandteilen verhindert.

Das Anwachsen der Mikroorganismenkultur (d. h. der Verfahrensschritt vor dem eigentlichen Kultivieren) kann unter Rieselbett- oder Wirbelbettreaktorführung erfolgen. Das Reaktionsmedium, das für das erfindungsgemäße Verfahren eingesetzt werden kann, ist vorzugsweise ausgewählt aus Wasser, Nährlösung (wie z. B. Czapek Dox Broth Medium) und Lösung mit umzusetzendem Substrat.
In Abhängigkeit von dem Reaktionsmedium und den Mikroorganismen wie filamentösen Pilzen (besonders Penicilli, Aspergilli, Fusarien, Trichodermen), Hefen und Bakterien umfassen die Reaktions-/Abbauprodukte der Mikroorganismenkulturen, Proteine einschließlich Enzymen und Antikörper, Sekundär-Stoffwechselprodukte, umgewandelten Substrate einschließlich Alkohole, Aldehyde, Ketone, organische Säuren, Alkaloide, Pigmente und Aromastoffe, geeinigte Abwässer oder umgewandelte Xenobiotika. Besonders bevorzugte Reaktions-/Abbauprodukte sind Enzyme (insbesondere Hydrolasen, Oxidasen, Transferasen) und Sekundärmetaboliten (insbesondere Alkaloide).

Das erfindungsgemäße Verfahren kann darüber hinaus die weitere Auftrennung, das Abtrennen von festen Partikeln und Mikroorganismen durch Filtrieren oder Sedimentieren und/oder Trennen mittels Ultrafiltration und/oder Chromatographie umfassen. Es ist weiterhin bevorzugt, dass bei Überschreitung eines Druckverlustes über der Befüllung des Fliessbetts von > 100 Pa/m, Schütthöhe oder Festbettfüllung, vorzugsweise > 50 Pa/m, von der Rieselbettreaktor-Verfahrensführung in die Wirbelbettreaktor-Verfahrensführung umgeschaltet wird.

Der Zeitraum, in dem die Wirbelbettreaktor-Verfahrensführung erfolgt, wird vorzugsweise so gewählt, dass bei der nachfolgenden Rieselbettreaktor-Verfahrensführung die Überschreitung des Druckverlustes zurückgeführt ist. Sie dauert vorzugsweise wenigstens 1 Minute, insbesondere 10 min bis 4 h. Die Wirbelbettverfahrensweise kann dabei auch dazu genutzt werden, dass hiermit gewünschte Produktions-, Stoffumwandlungs-, Abbaueffekte und Ablösungseffekte der Produkte vom Wirbelbett erreicht werden.

Das Kultivierungsverfahren der Erfindung kann weiterhin die Beaufschlagung von Druck umfassen, wobei das Reaktionsmedium durch unter dem Festbett gegebenenfalls vorhandene Sieb- und Filterböden gepresst wird. Diese Sieb- und Filterböden können dabei verschiedene Maschenweiten/ Porengrößen, insbesondere absteigend, abhängig vom Bettmaterial zwischen 1 mm bis zum Ultrafiltrationsbereich von ca. 5000 Dalton, aufweisen. Durch den Einsatz solcher Sieb- und Filterböden kann eine sukzessive Isolierung von Partikeln verschiedener Größenordnungen erzielt werden. Weiterhin bevorzugt ist dabei eine schräge Anordnung der Filter- und Siebböden, die es ermöglicht, die Produkte durch Abwaschen durch Wasser/Puffer einzeln zu eluieren und getrennt für die Weiterverarbeitung aufzufangen.

In dem Kultivierungsverfahren der Erfindung können vorzugsweise als Festbett alle möglichen organischen festen Substrate einschließlich Holz, Einjahrespflanzen, anderes Pflanzenmaterial, Humus, Kohle, organische Abfälle aller Art sowohl pflanzlicher als auch tierischer Herkunft als Einzelstoffe oder als Gemische für die Anzucht von Mikroorganismen-Reinkulturen oder Gemische eingesetzt werden. Ebenso können als Festbett Füllkörper, insbesondere solche aus Kunststoffen, Keramik, Glas und mineralischen Materialien, Aktivkohle, Kieselerde oder Schlacken eingesetzt werden, worauf die Mikroorganismenkulturen als Reinkulturen oder Gemische frei oder immobilisiert zum Wachsen gebracht werden.

Den auf dem Festbett befindlichen Mikroorganismen können zusätzlich Enzyme, bevorzugt Hydrolasen und/oder Oxidoreduktasen, frei oder immobilisiert (bevorzugt über das Reaktionsmedium) zugegeben werden. Es ist auch bevorzugt, beim Kultivieren dem Mikroorganismus Co-Enzyme zuzugeben, die frei oder immobilisiert oder mit dem jeweiligen Enzym co- immobilisiert sein können. Solche Co-Enzyme sind vorzugsweise ausgewählt aus NAD, NADP, FAD, CoA, PAL, TPP und ATP.

In einer weiteren bevorzugten Ausführungsform des Kultivierungsverfahrens wird beim Kultivieren eine Auftrennung, partielle Aufreinigung oder Konzentrierung der gebildeten Produkte oder umgewandelten Substrate erzielt. Es ist weiterhin besonders bevorzugt, dass bei der Rieselbettreaktor-Verfahrensführung unterschiedliche Organismen oder Organismen-Mischkulturen eingesetzt werden als bei der Wirbelbettreaktor-Verfahrensführung und insbesondere auch verschiedene Enzyme und Co-Enzyme den verschiedenen Reaktorfahrweisen zugegeben werden.
In einer besonders bevorzugten Ausführungsform wird das erfindungsgemäße Verfahren zur Produktion von Enzymen, zur Produktion von Sekundärmetaboliten, zur Behandlung von pflanzlichen Material, insbesondere Holz und Einjahrespflanzen, als Biokatalysatorsystem bei der chemischen Synthese, in der Schadstoffentfernung aus Abwasser und zum Abbau von Xenobiotika eingesetzt. Bei dem erfindungsgemäßen Verfahren können bei einer sukzessiven Produktion von Enzymen, Sekundärmetaboliten, chemischen Zwischen- oder Endprodukten in der Rieselbettreaktor-Verfahrensführung und/oder der Wirbelbettreaktor-Verfahrensführung die entsprechenden Produkte durch die vorhandenen Separierungsmöglichkeiten des Bioreaktors vorgereinigt werden.
Bei der Behandlung von pflanzlichem Material (Holz, Einjahrespflanzen), beim Einsatz in der Schadstoffentfernung aus Abwasser und beim Abbau von Xenobiotika ergibt sich durch die Möglichkeit der verschiedenen Verfahrensführung eine Verbesserung der Behandlungsleistung oder Abbauleistung.

In einer weiteren bevorzugten Ausführungsform erfolgt eine Reaktorsteuerung durch im Reaktor vorhandene Sensoren zur pH-, pO₂-Messung, zur Messung von Feuchte, Temperatur und Druck über Soll/Istwert-Regelung. Weiterhin können mehrere der Bioreaktoren in Serie geschaltet sein, so dass die Produkte des ersten Bioreaktors dem zweiten beziehungsweise den folgenden als Substrate zugeführt werden. Schließlich kann die bei der Wirbelbettreaktor-Verfahrensführung segmentierte Rieselbettkultur als Animpfkultur für die nächste Scale-up Stufe des Rieselbetts und/oder Wirbelbetts dienen, indem sie teilweise oder ganz in die nächste Scale-up Stufe überführt wird.

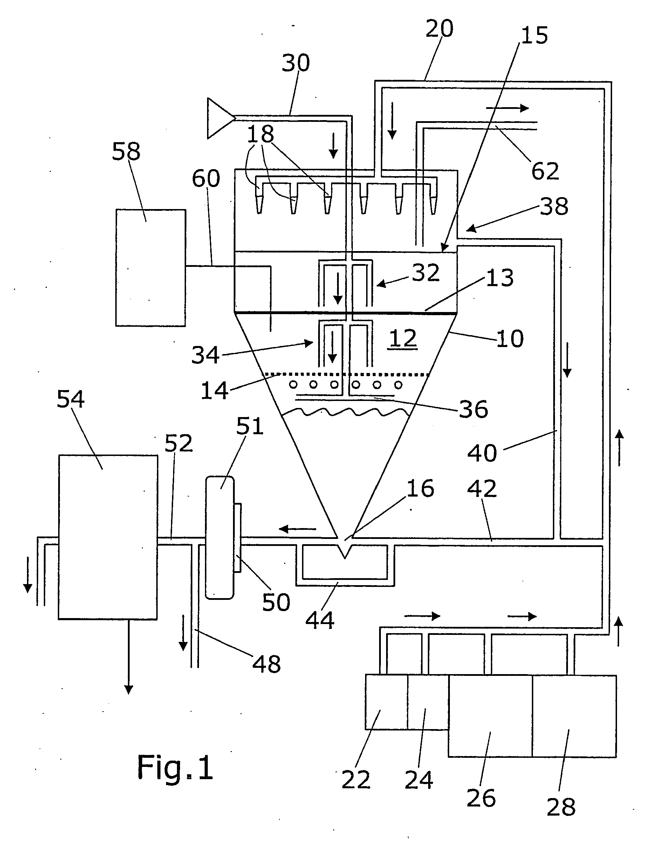

Bei einer ersten Ausführungsform des erfindungsgemäßen Bioreaktors (2) (Fig. 1 - 3) dessen Prinzip in Fig. 1 vereinfacht dargestellt ist, ist in einem Reaktionsbehälter 10 ein Festbett 12 angeordnet. Das Festbett 12 wird durch eine Festbettauflage 14, wie ein Gitter oder Sieb, gehalten und ist innerhalb des Reaktionsbehälters 10 angeordnet. Auf Grund der Formgebung des Reaktionsbehälters 10, der sich in Fig. 1 nach unten, d.h. in Richtung eines Auslasses 16, verjüngt, kann die Festbettauflage 14 auf einfache Weise in den im Querschnitt dreieckigen Reaktionsbehälter eingelegt werden. Eine gesonderte Befestigung oder Aufnahme für die Festbettauflage 14 kann vorgesehen sein. Es ist somit möglich, das Rückhalteelement 14 durch einfaches Herausheben aus dem Reaktionsbehälter zu entnehmen.

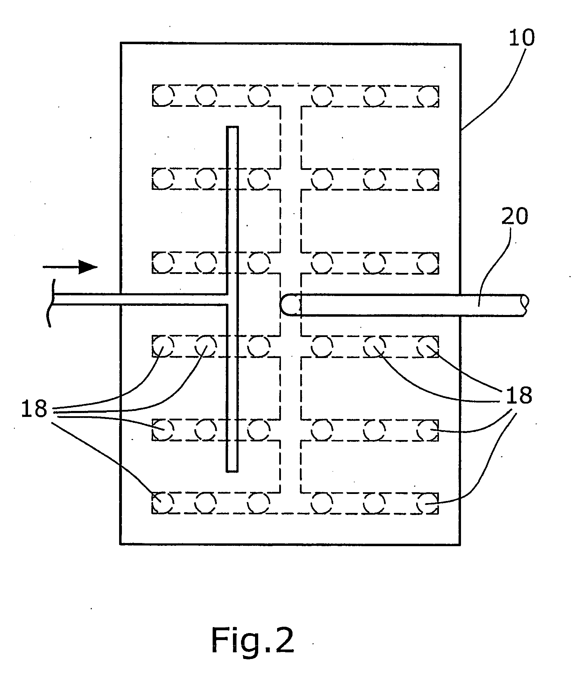

Oberhalb der in der Höhe variabel gestaltbaren Festbettoberfläche (beispielhaft 13) sind mehrere Rieseldüsen 18 angeordnet, die über eine gemeinsame Zuleitung 20 versorgt werden. Die einzelnen Rieseldüsen 18 sind von oben gesehen (Fig. 2) über den gesamten Querschnitt des Reaktionsbehälters 10 im Wesentlichen regelmäßig verteilt angeordnet. Die Rieseldüsen 18 sind über die Rohrleitung 20 mit unterschiedlichen Tanks 22, 24, 26, 28 verbunden. In diesen kann beispielsweise eine Säure, eine Lauge, Wasser, eine Pufferlösung und/ oder eine Nährstofflösung vorgesehen sein. Durch derartig angeordnete Pumpen und Ventile können diese Flüssigkeiten in vorgegebenen Mengen und/oder Verhältnissen über die Rieseldüsen 18 dem Reaktor 10 zugeführt werden.

Ferner ist eine Belüftungseinrichtung 30 mit mehreren Belüftungsdüsen 32, 34, 36 vorgesehen. Im dargestellten Ausführungsbeispiel sind die Düsen 32 (in Rieselbettverfahrensweise) oberhalb des Festbettes 12 angeordnet oder ragen nur geringfügig in das Festbett 12 hinein. Die Düsen 34 sind innerhalb des Festbettes 12 angeordnet und die Düsen 36 sind unterhalb des Festbettes 12 angeordnet. Durch die Belüftungseinrichtung 30 kann Luft oder ein anders Gas in den Reaktionsbehälter 10 eingebracht werden, um ein Verstopfen des Festbettes 12 zu vermeiden. Auch kann über die Belüftungseinrichtung 30 ein die Reaktion in dem Bioreaktor beeinflussendes Gas zugeführt werden.

Über eine Entnahmeeinrichtung 38, die geeignete Absperrventile und ggf. eine Pumpe aufweist, kann im dargestellten Ausführungsbeispiel oberhalb des Festbettes 12 Medium aus dem Reaktor 10 entnommen werden. Das Medium wird sodann über ein Rohr 40 abgeführt, wobei es möglich ist, das abgeführte Medium erneut über das Rohr 20 und die Rieseldüsen 18 dem Behälter 10 zuzuführen, bevorzugt aber für den Wirbelschichtbetrieb über Rohr 40, 42 durch 16 wieder dem Bioreaktor zuzuführen. Ferner ist es möglich, das über die Entnahmeeinrichtung 38 und das Rohr 40 abgeführte Medium über Rohrleitungen 42, 44, 48 beispielsweise zu entsorgen oder einer Aufbereitung oder Weiterbehandlung zuzuführen. Hierbei kann ggf. ein Filter 50 zwischengeschaltet sein. Über ein mit dem Rohr 44 verbundenes Rohr 52 kann das abgeführte Medium auch einem Vorratstank 54 zugeführt werden.

In dem spitz zulaufenden bzw. sich verjüngenden unteren Bereich des Reaktionsbehälters 10 wird das Reaktionsmedium 56 gesammelt. Das Reaktionsmedium 56 kann über den Auslass 16 und die Rohrleitungen 46, 52 dem Vorratstank 54 oder über die Rohrleitung 48 einem weiteren Prozess etc. zugeführt werden.

Zur Unterstützung des Lösens von Partikeln und dgl. innerhalb des Rückhalteelementes 14 und/oder des Festbettes 12 ist es möglich, den Bioreaktor 10 zu fluten. Hierzu wird die Förderrichtung des Fluids umgekehrt und beispielsweise aus dem Vorratstank 54 durch die Rohre 52, 42 und den sodann als Einlass dienenden Auslass 16 Reaktionsflüssigkeit 56 in den Bioreaktor gepumpt. Die Reaktionsflüssigkeit kann ggf. über die Entnahmeeinrichtung 38 abgeführt und über das Rohr 42 dem Bioreaktor 10 wieder zugeführt werden. Um, deutlich unterschiedlich zum Stand der Technik, ein Verblocken des Festbetts aufzulösen, kann nach dem Fluten durch 32 und/oder 34 und/oder 36 Druckluft in oder unter das Festbett gepumpt werden, um das besonders bei filamentösen Pilzen entstandene Mycelgeflecht zu zerstören und das Festbett so zu entblocken.

Des Weiteren weist der erfindungsgemäße Bioreaktor eine Messwerterfassungs-Einrichtung 58 auf, die mit einer oder mehreren Messsonden 60 verbunden ist. Die Messsonden 60 sind beispielsweise innerhalb des Festbettes 12 angeordnet und dienen beispielsweise zur Aufnahme des Feuchtigkeitsgehalts, des pH-Wertes etc.

Des Weiteren ist mit dem inneren des Bioreaktors ein Abluftrohr 62 verbunden, das ggf. über ein Ventil verschließbar ist und zur Regelung des innerhalb des Bioreaktors 10 herrschenden Drucks dient.

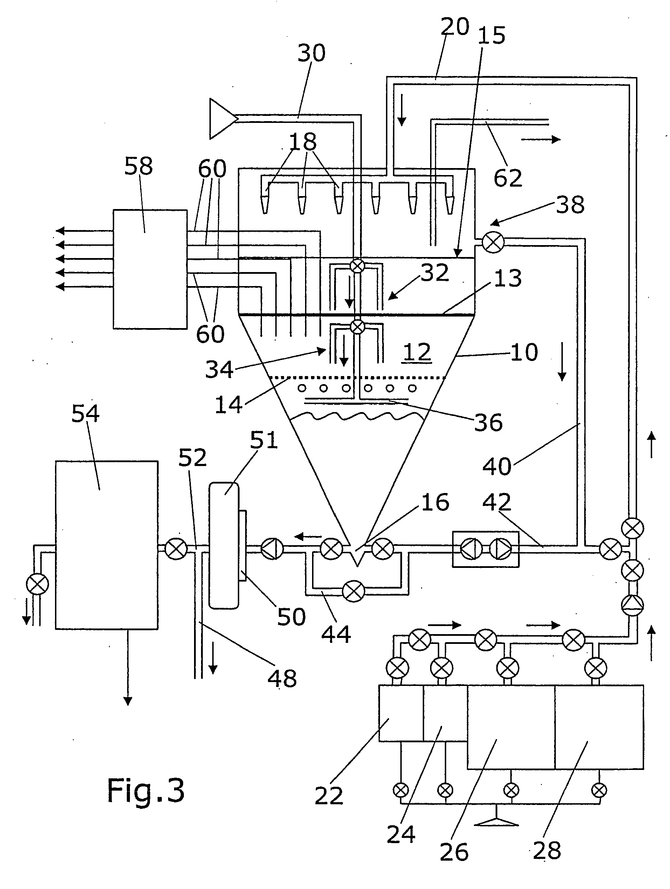

Fig. 3 entspricht prinzipiell der Fig. 1, wobei zusätzlich die erforderlichen Pumpen und Ventile schematisch dargestellt sind.

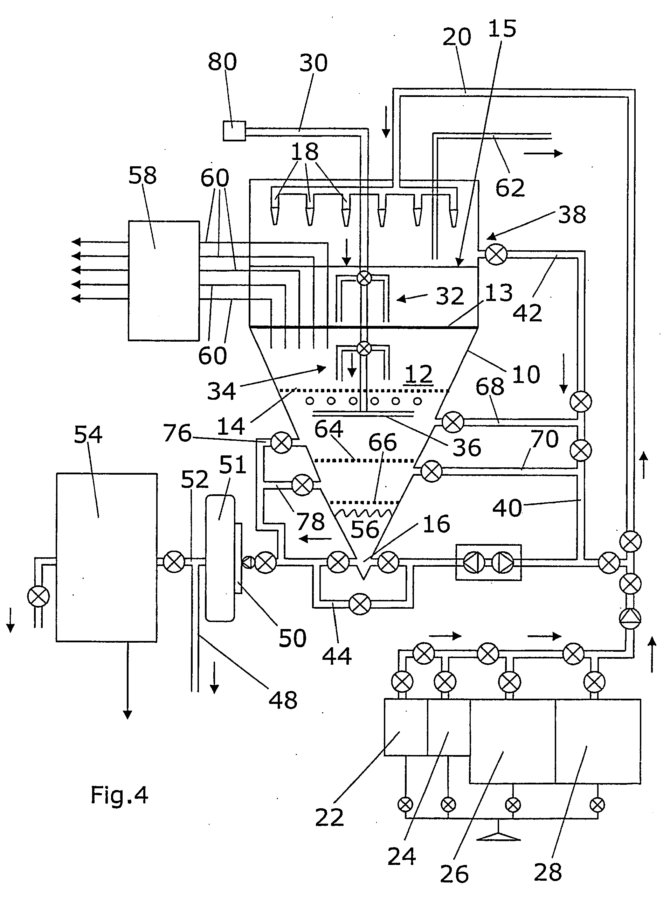

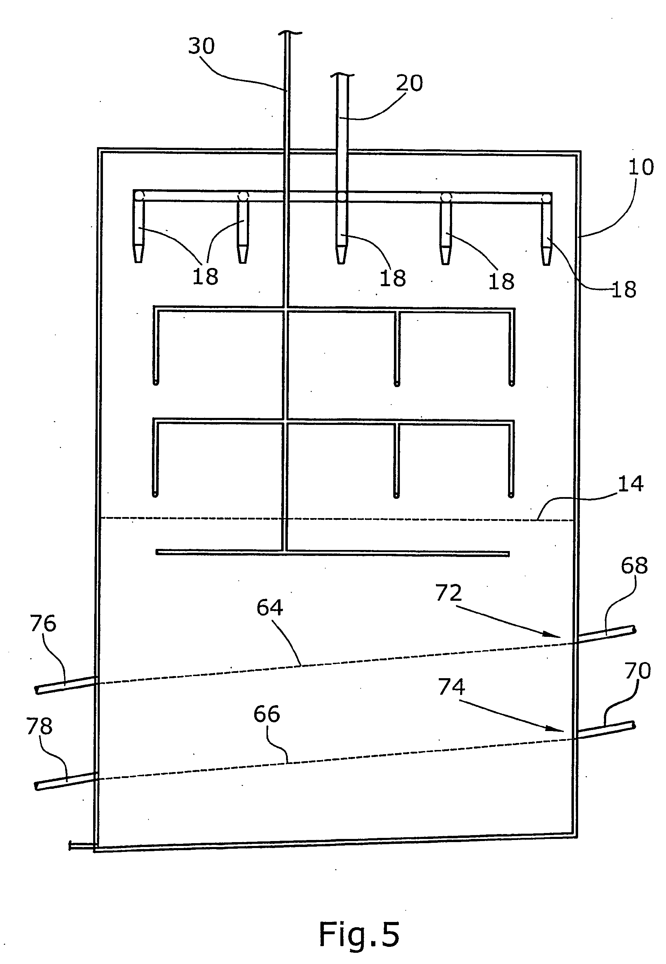

Die in den Fig. 4 und 5 dargestellte Ausführungsform der Erfindung stellt eine Erweiterung der anhand der Fig. 1 - 3 beschriebenen Erfindung dar. Identische oder ähnliche Bestandteile sind daher mit denselben Bezugszeichen bezeichnet.

Bei dieser Ausführungsform (Fig. 4 und 5) sind zusätzlich unterhalb des Rückhalteelements 14 Filterelemente 64, 66 vorgesehen. Die Filterelemente 64, 66 dienen zum Filtern des erzeugten Reaktionsmediums, wobei die Porengröße des Filterelements 66 kleiner ist, als die Porengröße des Filterelements 64.

Im dargestellten Ausführungsbeispiel sind die Filterelemente 64, 66 geneigt angeordnet (Fig. 5). Zur Produktentnahme sowie zur Reinigung der Filterelemente 64, 66 ist jeweils eine Spüleinrichtung 68, 70 vorgesehen. Mit dieser ist es möglich, über Einlässe 72, 74 eine Spülflüssigkeit auf die Filterelemente 64, 66 zu leiten. Auf der den Spüleinrichtungen 68, 70 gegenüberliegenden Seite des Bioreaktors 10 sind Abführeinrichtungen 76, 78 vorgesehen, durch die die Produkte und die Reinigungsflüssigkeit wieder abgeleitet werden kann. Wie aus Fig. 4 ersichtlich, kann als Spülflüssigkeit Medium über die Entnahmeeinrichtung 38 und das Rohr 42 entnommen und zum Spülen der Filtereinrichtung 64, 66 genutzt werden.

Des Weiteren ist eine Druckerzeugungseinrichtung 80 vorgesehen, die mit der Belüftungseinrichtung 30 verbunden ist. Durch die Druckerzeugungseinrichtung 80 kann innerhalb des Bioreaktors 10 ein Druck erzeugt werden, wobei der Druck oberhalb des Festbettes 12 höher ist, so dass Reaktionsmedium durch das Rückhalteelement 14 und die Filterelemente 64, 66 gedrückt werden kann. Hierdurch kann zusätzlich eine Reinigung des Rückhalteelements 14 sowie der Filterelemente 64, 66 erfolgen.

Die Filterelemente 64, 66 sind in den sich verjüngenden Teil des Bioreaktors 10 eingelegt. Hierzu können entsprechende Auflagen an der Innenwand des Bioreaktors vorgesehen sein. Ebenso ist es möglich, den Bioreaktor selbst in diesem Bereich stufenförmig auszubilden, so dass die Filterelemente auf entsprechende Stufen gelegt werden können.

Eine entsprechende Druckerzeugungseinrichtung kann auch in der anhand der Fig. 1 - 3 beschriebenen Erfindung vorgesehen werden.

Mit dem beschriebenen neuen Prinzip der Kombination eines Rieselbettreaktors mit dem Wirbelbettreaktor ist es möglich, alle möglichen organischen festen Substrate wie z.B. Holz, Einjahrespflanzen, anderes Pflanzenmaterial, Humus, Kohle, organische Abfälle aller Art sowohl pflanzlicher als auch tierischer Herkunft als Einzelstoffe oder als Gemische als Festsubstrat (Festbett) für die Anzucht von Mikroorganismen-Reinkulturen (Bakterien, Hefen, andere Pilze) oder Mischkulturen zu benutzen.

Dabei ist es auch möglich, dass den Kulturen zusätzlich Enzyme aus allen Klassen nach der "International Enzyme Nomenclature: Committee of the International Union of Biochemistry and Molecular Biology (Enzyme Nomenclature, Academic Press, Inc., 1992)", bevorzugt Hydrolasen und/oder Oxidoreduktasen zugegeben werden und diese als freie Enzyme oder immobilisiert zugegeben werden. Dabei kann die Immobilisierung nach allen gängigen Methoden des Standes der Technik wie z.B. kovalente Bindung, adsorptive Bindung, Kreuz-verknüpfung an Trägermoleküle oder Carrier, durch Matrixeinbindung in z. B. Polysaccharidmatrices (Algin, etc.), Copolymerisation von Enzymen an Zellen und durch Mikroverkapselung durchgeführt werden.

Zusätzlich ist es möglich, dass frei oder immobilisiert oder mit dem jeweiligen Enzym co-immobilisiert vorliegende Co-Enzyme zugegeben werden.

Des Weiteren ist es möglich, dass die Kulturen auf Füllkörpern (Carriern) (hauptsächlich ringförmig, rund) aus z. B. Kunststoffen, Keramik oder Glas und mineralischen Materialien wie z. B. Aktivkohle, Kieselerde oder Schlacken etc. aufgebracht werden und frei oder immobilisiert zum Wachsen gebracht werden, wobei die Nährstoffzufuhr hauptsächlich über die Flüssigkeitsversorgung bereit gestellt wird. Auch hier ist eine zusätzliche Zugabe von Enzymen und/oder Co-Enzymen frei, immobilisiert oder co-immobilisiert möglich.

Dabei ist es unter Umständen nicht nötig, dass bei der Fahrweise als Wirbelbettreaktor die organismenbewachsenen Substrate oder Carrier eine Dichte besitzen über der Dichte von Wasser, um durch den Auftrieb in Schwebe gehalten zu werden, da durch ein Auf- und Absinken der bewachsenen Substrate/Carrier eine weitere gewünschte Loslösung von Organismen erfolgt.

Ein weiteres wichtiges Charakteristikum der vorliegenden Erfindung ist die Möglichkeit der während der Kulturdauer stattfindenden weiteren Auftrennung/Aufreinigung der gebildeten Produkte wie z.B. Enzyme, Stoffwechselprodukte oder umgewandelten, d.h. chemisch veränderten Substrate.

Dies wird erfindungsgemäß dadurch gelöst (Fig. 1 - 3), dass a) (abhängig von der Aufgabenstellung und dem Druckverhalten des bewachsenen Rieselfestbettes) durch Druck oder Abpumpen die unter dem Rieselfestbett aufgefangene Flüssigkeit durch eine Vorfiltereinheit und Sterilfiltrationseinheit in ein Zusatzgefäß gepumpt wird und entweder nach Messung der kulturrelevanten Produktparameter die Lösung weiterverarbeitet wird (Ultrafiltration/ Flüssigchromatographie/ Analytik etc.) oder bei nicht ausreichender Produktqualität und/oder Konzentration in die Fermentationseinheit über die Rieseleinheit zurückgeführt wird. Die entsprechenden Messungen im Zusatzgefäß können manuell nach Probenentnahme oder kontinuierlich durch FIA (flow injection analysis) erfolgen. Die aufgefangene Flüssigkeit kann auch direkt ohne Durchgang durch ein Zusatzgefäß nach Partikel- und Sterilfiltration über ein Ultrafiltrationsmodul eingeengt werden.

Dies wird ebenfalls erfindungsgemäß dadurch gelöst (Fig. 4 und 5), dass b) (abhängig von der Aufgabenstellung und dem Druckverhalten des bewachsenen Rieselfestbettes) speziell unter dem Rieselbett Siebböden und Filterböden angeordnet werden, durch die optional mit Druckbeaufschlagung das Medium durch spezielle Siebe bzw. Filter gepresst werden kann, welche verschiedene Maschenweiten/ Porengrößen (absteigend, abhängig vom Bettmaterial zwischen 1 mm bis zum Ultrafiltrationsbereich von ca. 5000 Dalton) haben und eine sukzessive Isolierung von Partikeln verschiedener Größenordnungen erlauben.

Durch die schräge Anordnung der Filter und/ oder Siebe 64, 66 können die Produkte durch seitliches Einspritzen von Wasser/Puffer einzeln eluiert und getrennt für die Weiterverarbeitung aufgefangen werden. Dies dient zusätzlich der Reinigung der Filter. Ein Verstopfen der Filter wird zusätzlich behoben, indem von unten Medium in den Bioreaktor 10 eingebracht wird und die Filter 64, 66 unter Druck von unten freigespült werden. Nichtumgesetzte Substrate, die sich über den Filtern 64, 66 ablagern, können nach dem Spülen wieder dem Reaktor 10 zugeführt werden.

Diese neue Verfahrensführung ist von sehr großer Wichtigkeit, da sie die sukzessive Aberntung von z.B. Enzymen erlaubt. So ist z.B. beschrieben, dass lignolytische Enzyme bei vielen Weißfäulepilzen z.B Phanerochaete chrysosporium, Trametes versicolor u.a. auf vielen Nährböden und auch beim Wachstum auf festen Substraten nacheinander gebildet werden z.B. in der Reihenfolge: Mangan-Peroxidase → Laccase (z.B. bei Trametes).

Es wäre von erheblichem Vorteil, die verschiedenen Enzymspezies zumindest mit wesentlich weniger Kontamination durch andere Begleitenzyme erhalten zu können. Dies ist zum ersten Mal mit Hilfe dieser Fermenterfahrweise in erheblichen Ausbeuten möglich. Die Kulturführung kann als Batchverfahren, als Fedbatch-Verfahren oder kontinuierlich erfolgen (abhängig vom Trägermaterial und der Fragestellung). Als Prozesssteuerungsparameter können pH, Temperatur, pO₂, Feuchte, Biomasse und Druck gemessen werden.

Ein weiteres Merkmal der vorliegenden Erfindung ist, dass man durch Untereinanderschaltung oder Nebeneinanderschaltung (mit verschiedenen Breiten- und/oder Höhenabmessungen der Reaktorgefäße) des in Abbildung I dargestellten Bioreaktoraufbaus zu einem Mehrfach-Bioreaktorsystem kommen kann, bei dem durch die unterschiedlichen Volumina fassenden Rieselreaktor-Festbette und entsprechenden Wirbelbette eine völlig neue Möglichkeit des Scale-up dieser Reaktorfahrweise möglich wird. So können mehrere Reaktoren parallel geschaltet werden, in denen sich Festbette und Wirbelbette abwechseln und die gebildeten Produkte des einen vom anderen in einem kontinuierlichen Verfahren weiterverarbeitet werden. Es kann ebenfalls nach der Fließbett-/Wirbelschichtfahrweise die durch diese Fahrweise segmentierte Rieselbettkultur als Animpfkultur für die nächste Scale-up Stufe des Rieselbetts (und oder Wirbelbett) dient, in dem sie teilweise oder ganz in die nächste Scale-up Stufe überführt wird.

### Detailliertere Beschreibung der Fermenterfunktion

Die erfindungsgemäße Bauweise, wie z. B. in Fig. 1 - 3 gezeigt, erlaubt eine sehr gute Regelung der Feuchte, was oft ein sehr großes Problem bei Festbettreaktoren darstellt.

Über die Rieseldüsen 18 kann Nährlösung aus dem Nährlösungstank 28 oder Wasser/Puffer über den Wasser- bzw. Puffertank 26 auf das Festbett 12 gepumpt werden.

Ebenso kann zur pH-Einstellung bzw. pH-Nachstellung Säure aus dem Tank 24 oder Lauge aus dem Tank 22 über die Rieseldüsen 18 zugegeben werden. Die Luftzufuhr erfolgt über die Belüftungseinrichtung 30. Die Abluft wird über den Auslass 62 abgeleitet.

Die aus dem Festbett 12 austretende Flüssigkeit 56 gelangt in einen Bodentank und kann über die Rieseldüsen 18 über eine Leitung 42, 20 wieder auf das Festbett 12 zurückgeführt werden.

Die Flüssigkeit 56 aus dem Bodentank kann auch über eine Pumpe durch den Partikelfilter 50 und dem Sterilfilter 51 in den Vorratstank 54 gepumpt werden. Bei eventuell erforderlichem höheren Druck für die Filtrationen erfolgt dies durch die Druckstutzen 32.

Die Flüssigkeitsparameter im Vorratstank 54 können manuell gemessen werden oder z.B. durch FIA automatisch bestimmt werden. Optional ist eine sofortige Einengung der Flüssigkeit mittels Ultrafiltration vor der Befüllung des Vorratstanks 54 möglich.

Über die Messwerterfassung 58 werden pH, pO₂ Temperatur, Feuchte und Druck außerhalb des oder im Reaktorbett 12 durch entsprechende Sensoren 60 erfasst, und die Zugabe von Säure/Base, Sauerstoffzufuhr, Temperatureinstellung (doppelwandiger temperierter Reaktormantel, gesteuerte Luftpulse, temperierte Berieselung), Feuchte (Berieselungsmenge bei mangelnder Feuchte oder Zufuhr trockener Luft bei zu hoher Feuchte) und Druck (Änderung der Verfahrensführung: Rieselbett → Wirbelbett) geregelt.

Bei Änderung der Verfahrensführung von Rieselbett zu Wirbelbett wird durch Luft über Druckerhöhung über die Belüftungszufuhr 30, 32, 34, 36 bei ggf. gleichzeitiger Flutung des Festbettes 12 mit (optional) Nährlösung/ Wasser/Puffer das Festbett 12 aufgelöst und über die Luftzufuhr 30, 32, 34, 36 die Wirbelbettverfahrweise aufrechterhalten. Über die Leitung 40 wird die Flüssigkeit im Kreislauf gefahren. Durch Beendigung der Belüftung, Beendigung der Flüssigkeitszufuhr über den Bodentank und Starten der Berieselung über die Rieseldüsen 18 kann die Rieselbettfahrweise nach optionaler Trocknung des Bettes 12 mittels der Belüftungseinrichtung 30 erneut gestartet werden.

Die in Fig. 4 und 5 dargestellte Bioreaktorvariante mit Sieb- und Filterkompartimentierung unter dem Festbett 12 erlaubt die Möglichkeit der Partikeltrennung in der durch das Festbett geleiteten Flüssigkeit. Dazu sind Siebe/ Filter 64, 66 unter dem Festbett 12 eingebaut. Eine Entleerung der einzelnen Stufen ist über die Ausgänge 76 oder 78 (Bodentank) durch Druckbeaufschlagung oder Abpumpen möglich. Ein Verstopfen der Filter 64, 66 wird dadurch gelöst, dass a) die Filter 64, 66 freigespült werden, und/ oder (b) dass die Kompartimente unterhalb der Filter 64, 66 kontrolliert sukzessiv geflutet werden und die Filter von unten unter Druck freigespült werden.

Die Erfindung wird durch die folgenden Beispiele näher erläutert, soll aber nicht auf diese beschränkt sein.

### Beispiele

### Beispiel 1: Produktion und sukzessive Gewinnung von lignolytischen Enzymen mittels Kombination aus Wirbelbettreaktor.

Der Reaktor mit einem Festbett-Volumen von ca. 8 I wird mit Zellstoff (Softwood/ Kraft) in solcher Weise befüllt, dass nach Durchmischen mit einer mit Hilfe eines Waring blendor's s gemixten Standkultur von Trametes versicolor und pH-Einstellung auf pH 4,5 ca. 4 - 6 % Stoffdichte resultiert. Es wird soviel Zellstoff/Inokulummenge auf die Siebplatte (13, Fig. 1 - 3) gegeben, dass eine Festbetthöhe von ca. 5 - 8 cm erhalten wird. Das Verhältnis Zellstoff/Inokulum beträgt ca. 30:1 bis 10:1. Die Kultur wird mit Beginn der Berieselung und Belüftung gestartet. Die Berieselungsflüssigkeit enthält 0,05 bis 0,5 % Glukose und 0,01 bis 0,1 % Stickstoff in Form von Asparagin bei pH 4,5.

Die Ablaufflüssigkeit wird nach Überführung in das Vorratsgefäß nach Laccase und Mangan-Peroxidase detektiert.
Nach ca. 3 - 5 Tagen wird zunächst die Mangan-Peroxidase und zwischen Tag 5 - 8 die Laccase geerntet, die durch diese sukzessive Aberntung nur ca. 10 - 20 % mit dem jeweils anderen Enzym verunreinigt ist.
Nach ca. 10 Tagen Kulturdauer ist eine relativ starke Verblockung des Fettbetts (> 100 Pa/ m Festbett) festzustellen.

Um diese Verstopfung zu beheben und auch durch das Ändern der Kultur-Fahrweise eine Vereinzelung (Zerkleinerung) der gebildeten Mycelmatte zu erreichen, wird durch Druckbeaufschlagung über die Belüftungseinrichtung 30 bei gleichzeitiger Flutung des Festbettes 12 und Beendigung der Berieselung von der Rieselbettreaktor-Fahrweise auf die Wirbelbett-Fahrweise umgeschaltet, dergestalt, dass das Medium von unten in den Kreislauf gepumpt wird und die Belüftung über die Belüftungseinrichtung 30 so eingestellt wird, dass auch ohne Abdeck-Filter kein bewachsener Zellstoff ausgetragen wird. Es wird eine starke Segmentierung des Mycels erhalten, welches nach Beendigung der Wirbelbett-Fahrweise nach ca. 4 Stunden und erneutem Start der Rieselbettreaktor-Fahrweise (Drosselung der Belüftung, nur noch Belüftung, Anschalten der Berieselung) zu einem guten Anwachsen der Kultur führt.

Nach ca. 3 - 5 Tagen Kulturdauer kann wieder eine Ernte von zunächst Mangan-Peroxidase und zwischen Tag 5 - 8 eine Ernte von Laccase erhalten werden, die entsprechend der ersten Aberntung ebenfalls nur ca. 10 - 20 % mit dem jeweils anderen Enzym verunreinigt ist. Insgesamt sind ca. 4 Zyklen mit relativ guten Ausbeuten möglich.

Bei nötiger steriler Fahrweise wird das Aufschlag-Gefäß des Waring Blendor' s dampfsterilisiert und die zum Mischen von Kultur/Inokulum, Einstellen des pHs etc. benutzten Teile eines Starmixers oder eines Hobartmixers, die mit dem Mixgut in Berührung kommen mit Ethanol (75%ig) oder 2 M NaOH über Nacht sterilisiert. Mixen, Mischen und das Überführen des Mixgutes in das Festbett des Fermenters können unter einer sterilen Werkbank erfolgen.

Beispiel 2: Die Nutzung des Wirbelbettmodus ist nicht auf die Entblockung des Festbetts beschränkt. Anders als vom Stand der Technik bekannt, kann das Medium in der erfindungsgemäßen Ausführung des Bioreaktors im Kreislauf gepumpt und externen belüftet (mit O₂-angereichert) werden. So ist es möglich, Mikroorganismen zunächst im aeroben Wirbelschichtbetrieb auf einem Träger (z. B. Polyurethan, Perlite, Sinterglas, Biopolymere) scherkraftarm anwachsen zu lassen, um im darauf folgenden Rieselfilm-Betrieb von den Mikroorganismen sekretierte Produkte (z.B. Enzyme, Sekundärmetabolite, organische Säuren) abzuernten.

Verfahren A: Eine Conidiosporenlösung (10⁵ Sporen/ml) von *Aspergillus niger* wird im Wirbelschichtmodus, in einer 14 % Glucose, 0,04 % (NH₃)x(NH₃SO₄), 0,25 % KH₂PO₄, 0,05 % MgSO₄ 7 H₂O, 0,001 % FeSO₄, 0,00025 % ZnSO₄, pH 5,5) enthaltenden Lösung, langsam im Kreislauf gepumpt. Im Bioreaktor (10 Liter Festbettvolumen) besteht ein Wirbelbett aus Pulyurethan-Stücken (Kantenlänge 0,2 - 1 cm). Die Sporen setzen sich nach einigen Stunden auf dem Polyurethan-Träger fest. Wenn die Zahl der freien Sporen unter 10³/ml gesunken ist, wird das weiter im Kreislauf gefahrene Medium in einer externen Blasensäule belüftet um das schonende Anwachsen des Pilzes zu begünstigen. Nach vollständigem Anwachsen des Pilzes (1 - 2 Tage) wird das Verfahren in den Rieselfilmmodus umgestellt. Der angewachsene Pilz beginnt auf dem Polyurethan zu sporulieren und Zitronensäure zu bilden. Die Zitronensäure wird in definierten Abständen (10 - 30 h) durch die Rieseldüsen mit 200 ml vom Festbett abgewaschen. In nicht optimierten Aspergillus-Stämmen werden so Zitronensäurekonzentrationen bis 3 % in der Rieselflüssigkeit erhalten. Optional kann die Rieselflüssigkeit im Kreislauf gepumpt werden bis Citratkonzentrationen um 5 % erreicht werden.
Nach dem bereits bekannten Verblocken des Festbetts, dass unter diesen Bedingungen nach ca. 14 Tagen auftritt, wird erneut auf den Wirbelschichtmodus umgestellt und das Festbett durch den Flüssigkeitsstrom, unterstützt durch Druckluftpulse, durchwirbelt. Durch dieses Verfahren zerreißen, anders als im Stand der Technik bekannt, die die Pulyurethan-Stücke verbindenden Hyphen des Pilzes durch die starke Verwirbelung des Festbetts.

Verfahren B: Eine Conidiosporenlösung (10⁵ Sporen/ml) von *Aspergillus oryzae* wird im Wirbelschichtmodus, in einer 2 % Glucose, 1 % Pepton, 1 % Yeast Extract, 0,6 % Na-Acetat Lösung, 0.2 % MgSO₄ 7 H₂O 0.01 % KH₂PO₄, 0,05 % KCI, 0,01 % MnSO₄ · 5 H₂O, 0,01 % FeSO₄ · 7 H₂O 0,01 % NaCl-Lösung langsam im Kreislauf gepumpt. Im Bioreaktor (8 Liter Festbettbettvolumen) besteht ein Wirbelbett aus Perlit-Kugeln (Durchmesser 0,1 - 0,5 cm). Die Sporen setzen sich nach einigen Stunden in den Poren des inerten Träger fest. Wenn die Zahl der freien Sporen unter 10³/ml gesunken ist, wird das weiter im Kreislauf gefahrene Medium in einer externen Blasensäule belüftet um das Anwachsen des Pilzes zu begünstigen. Nach vollständigem Anwachsen des Pilzes (2 Tage) wird das Verfahren in den Rieselfilmmodus umgestellt. Durch die Rieseldüsen werden dünnflüssige Lipide (z. B. Olivenöl, Sojaöl) auf das Festbett geprüht. Der Pilz reagiert mit der Produktion des Enzyms Lipase, welches in 24 - 48stündigen Abständen täglich durch 300 ml Pufferlösungen (z. B. Phosphatpuffer 50 mM, pH 7) im Rieselfilmverfahren vom Festbett gespült wird. Pro ml Puffer können über 30 U Lipase gewonnen werden. Ein Verblocken des Festbetts nach ca. 10 Tagen wird durch die starken Scherkräfte, die nach Medienstromumkehr und Druckluftzufuhr zwischen den Trägermaterialien entstehen aufgelöst.

Verfahren C: Eine homogenisierte Mycellösung eines Sekundärmetabolitsekretierenden (z.B. Antibiotika, Mycotoxine, Pigmente) Ascomyceten, z.B. *Penicillium chrysogenum,* wird so lange im Wirbelschichtmodus durch das Wirbelbett (Volumen 1 L, bestehend aus einer Mischung aus Holzspänen, Roggen, Lignozellulose (Gewichtsverhältnis 1:1:2)) gepumpt und dabei extern belüftet, bis in der Animpflösung mikroskopisch kein Mycel mehr sichtbar ist. Durch das scherkraftarme Rieselfilmverfahren wächst der Pilz bei externer Belüftung an. Zur Produktion der Metabolite wird auf den Rieselfilmmodus umgestellt. Das Festbett wird durch die im Reaktor befindliche Belüftungseinrichtung angetrocknet, um bei Bedarf einen für Pilze günstige geringere Wasseraktivität des Festbettes einzustellen.
Die Metabolite werden über die durch die Rieseldüsen gegebenen Puffer oder Lösungsmittel vom Festbett abgewaschen. Ein Verblocken des Festbetts nach ca. 8 Tagen wird durch die starken Scherkräfte, die nach Medienstromumkehr und Druckluftzufuhr zwischen den Trägermaterialien entstehen aufgelöst.

## Patentansprüche

1. Kultivierungsverfahren für Mikroorganismen, umfassend eine Kombination aus Verfahrensschritten, die für einen Rieselbettreaktor charakteristisch sind, mit solchen, die für einen Wirbelbettreaktor charakteristisch sind, wobei
(a) Mikroorganismenkulturen auf einem Festbett unter Rieselbettreaktor-Verfahrensführung kultiviert werden, wobei das Reaktionsmedium durch Rieseldüsen aufgebracht wird, die Belüftung von unterhalb und/oder innerhalb des Festbetts erfolgt und die durch die Festbettschicht ablaufende Lösung, welche die Reaktions-/Abbauprodukte der Mikroorganismenkulturen enthält, entweder solange, bis die gewünschte Produktivität, das gewünschte Produkt bzw. das gewünschte Abbauprodukt oder der gewünschte Abbaugrad erreicht sind, zurückgeführt wird oder die abgelaufene Lösung unmittelbar zur weiteren Auftrennung weiterbehandelt wird und
(b) bei Überschreiten eines bestimmten Druckverlustes über die Befüllung des Festbetts durch Einleiten von Wasser oder Nährlösung und durch Einleiten von Luft die Kulturführung für einen gewissen Zeitraum von der Rieselbettreaktor-Verfahrensführung in eine Wirbelbettreaktor-Verfahrensführung umgeschaltet wird.

2. Kultivierungsverfahren nach Anspruch 1, wobei
(i) die Mikroorganismenkulturen ausgewählt sind aus Bakterien, Hefen, Pilzen, tierischen oder pflanzlichen Zellen und immobilisierten pflanzlichen und tierischen Geweben und insbesondere filamentöse Pilze sind; und /oder
(ii) bei der Wirbelbettverfahrensweise das Medium im Kreis gepumpt wird um ein Überlaufen von bewachsenem Füllkörper oder bewachsenen Festbettbestandteilen zu verhindern; und/oder
(iii) das Anwachsen der Mikroorganismenkultur unter Rieselbett- oder Wirbelbettreaktorführung erfolgt; und/oder
(iv) das Reaktionsmedium ausgewählt ist aus Wasser, Nährlösung und Lösung mit umzusetzendem Substrat; und/oder
(v) die Reaktions-/Abbauprodukte der Mikroorganismenkulturen Proteine einschließlich Enzymen und Antikörper, Sekundär-Stoffwechselprodukte, umgewandelte Substrate einschließlich Alkohole, Aldehyde, Ketone, Alkaloide, organischer Säuren, Pigmente und Aromastoffe, gereinigte Abwässer oder umgewandelte Xenobiotika sind und insbesondere Proteine und Sekundärmetaboliten sind; und/oder
(vi) die weitere Auftrennung das Abtrennen von festen Partikeln und Mikroorganismen durch Filtrieren oder Sedimentieren und/oder Trennen mittels Ultrafiltration oder Chromatographie umfasst; und/oder
(vii) bei Überschreitung eines Druckverlustes über der Befüllung des Fliessbetts von > 100 Pa/m Schütthöhe oder Festbettfüllung, vorzugsweise von > 50 Pa/m, von der Rieselbettreaktor-Verfahrensführung in die Wirbelbettreaktor-Verfahrensführung umgeschaltet wird; und/oder
(viii) der Zeitraum, in dem Wirbelbettreaktor-Verfahrensführung erfolgt, so gewählt ist, dass bei der nachfolgenden Rieselbettreaktor-Verfahrensführung die Überschreitung des Druckverlustes zurückgeführt ist, vorzugsweise wenigstens 1 Minute, insbesondere 10 min bis 4 h dauert, und/oder die durch diese zusätzliche Fahrweise gewünschten Produktions-, Stoffumwandlungs-, Abbaueffekte erreicht werden.

3. Kultivierungsverfahren nach Anspruch 1 oder 2, wobei
(i) das Verfahren das Beaufschlagung von Druck umfasst, wobei das Reaktionsmedium durch unter dem Festbett gegebenenfalls vorhandene Sieb- und Filterböden gepresst wird; und
(ii) die Sieb- und Filterböden von (i) verschiedene Maschenweiten/Porengrößen, insbesondere absteigend, abhängig vom Bettmaterial zwischen 1 mm bis zum Ultrafiltrationsbereich von ca. 5000 Dalton, aufweisen, was eine sukzessive Isolierung von Partikeln verschiedener Größenordnungen erlaubt; und
(iii) eine schräge Anordnung der Filter- und Siebböden von (i) und (ii) es ermöglicht, die Produkte durch Abwaschen durch Wasser/Puffer einzeln zu eluieren und getrennt für die Weiterverarbeitung aufzufangen.

4. Kultivierungsverfahren nach einem oder mehreren der Ansprüche 1 bis 3, wobei
(i) als Festbett alle möglichen organischen festen Substrate einschließlich Holz, Einjahrespflanzen, anderes Pflanzenmaterial, Humus, Kohle, organische Abfälle aller Art sowohl pflanzlicher als auch tierischer Herkunft als Einzelstoffe oder als Gemische für die Anzucht von Mikroorganismen-Reinkulturen oder Gemische eingesetzt werden; und/oder
(ii) als Festbett Füllkörper, insbesondere solche aus Kunststoffen, Keramik, Glas und mineralischen Materialien, Aktivkohle, Kieselerde oder Schlacken eingesetzt werden, worauf die Mikroorganismenkulturen als Reinkulturen oder Gemische frei oder immobilisiert zum Wachsen gebracht werden; und/oder
(iii) bei der Rieselbettreaktor-Verfahrensführung unterschiedliche Organismen oder Organismen-Mischkulturen eingesetzt werden als bei der Wirbelbettreaktor-Verfahrensführung; und/oder
(iv) den Mikroorganismen des Festbetts zusätzlich Enzyme, bevorzugt Hydrolasen und/oder Oxidoreduktasen, frei oder immobilisiert zugegeben werden; und/oder
(v) beim Kultivieren Co-Enzyme zugegeben werden, die frei oder immobilisiert oder mit dem jeweiligen Enzym co-immobilisiert sein können; und/oder
(vi) die Co-Enzyme von (v) vorzugsweise ausgewählt sind aus NAD, NADP, FAD, CoA, PAL, TPP und ATP.

5. Kultivierungsverfahren nach einem oder mehreren der Ansprüche 1 bis 4, wobei
(i) beim Kultivieren eine Auftrennung, partielle Aufreinigung oder Konzentrierung der gebildeten Produkte oder umgewandelten Substrate erfolgt; und/oder
(ii) bei der Rieselbettreaktor-Verfahrensführung unterschiedliche Organismen oder Organismen-Mischkulturen eingesetzt werden als bei der Wirbelbettreaktor-Verfahrensführung und insbesondere auch verschiedene Enzyme und Co-Enzyme den verschiedenen Reaktorfahrweisen zugegeben werden; und/oder
(iii) das Verfahren zur Produktion von Enzymen, zur Produktion von Sekundärmetaboliten, zur Behandlung von pflanzlichen Material, insbesondere Holz, Einjahrespflanzen, als Biokatalysatorsystem bei der chemischen Synthese, in der Schadstoffentfernung aus Abwasser und zum Abbau von Xenobiotika einsetzbar ist; und/oder
(iv) bei einer sukzessiven Produktion von Enzymen, Sekundärmetaboliten, chemischen Zwischen- oder Endprodukten in der Rieselbettreaktor-Verfahrensführung und/oder der Wirbelbettreaktor-Verfahrensführung die entsprechenden Produkte durch die vorhandenen Separierungsmöglichkeiten des Bioreaktors vorgereinigt werden; und/oder
(v) bei der Behandlung von pflanzlichem Material, insbesondere von Holz und Einjahrespflanzen, beim Einsatz in der Schadstoffentfernung aus Abwasser und beim Abbau von Xenobiotika die Möglichkeit der verschiedenen Verfahrensführung eine Verbesserung der Behandlungsleistung oder Abbauleistung ermöglicht.

6. Kultivierungsverfahren nach Anspruch 1, wobei
(i) durch im Reaktor vorhandene Sensoren zur pH-, pO₂-Messung, zur Messung von Feuchte, Temperatur und Druck eine Reaktorsteuerung über Soll/Istwert-Regelung erfolgt; und/oder
(ii) mehrere der Bioreaktoren in Serie geschaltet sind, bei denen die Produkte des ersten Bioreaktors dem zweiten beziehungsweise den folgenden als Substrate zugeführt werden; und/oder
(iii) die bei der Wirbelbettreaktor-Verfahrensführung segmentierte Rieselbettkultur als Animpfkultur für die nächste Scale-up Stufe des Rieselbetts und/oder Wirbelbetts dient, indem sie teilweise oder ganz in die nächste Scale-up Stufe überführt wird.

7. Bioreaktor zur Kultivierung von Mikroorganismen mit
einem Reaktionsbehälter (10),
einer in dem Reaktionsbehälter (10) angeordneten Festbettauflage (14) zum Anordnen eines Festbettes (12) auf der Festbettauflage (14),
oberhalb der Festbettauflage (14) angeordneten Rieseldüsen (18) zur Berieselung des Festbettes (12) mit Nährstofflösung, so dass der Bioreaktor im Rieselbettverfahren betrieben werden kann,
einer Belüftungseinrichtung (30, 32, 34, 36) zur Belüftung des Festbettes (12) bzw. des Innenraums des Reaktors (10), so dass der Bioreaktor im Wirbelbettverfahren betrieben werden kann, und
einer Messwerterfassung (58) zur Erfassung des Drucks außerhalb des oder im Festbett (12).

8. Bioreaktor nach Anspruch 7 mit
einem Reaktionsbehälter (10),
einer in dem Reaktionsbehälter (10) angeordneten Festbettauflage (14) zum Anordnen eines Festbettes (12) auf der Festbettauflage (14),
oberhalb der Festbettauflage (14) angeordneten Rieseldüsen (18) zur Berieselung des Festbettes (12) mit Nährstofflösung, so dass der Bioreaktor im Rieselbettverfahren betrieben werden kann und
einer mit dem Reaktionsbehälter (10) verbundenen Druckerzeugungseinrichtung (80) zum Hindurchpressen von Reaktionsmedium (56) durch die Festbettauflage (14).

9. Bioreaktor nach Anspruch 7 oder 8 der zur Durchführung eines Verfahrens nach einem der Ansprüche 1 - 6 geeignet ist.

10. Bioreaktor nach einem oder mehreren der Ansprüche 7 - 9, **gekennzeichnet durch** eine oberhalb der Festbettauflage (14) angeordnete Rückhaltevorrichtung (15) für die Wirbelbettreaktionsführung und/oder durch eine oberhalb der Festbettauflage (14) und oberhalb der Rückhalteelemente (15) angeordnete Entnahmeeinrichtung (38) zur Mediumabfuhr, insbesondere in geflutetem Zustand.

11. Bioreaktor nach einem oder mehreren der Ansprüche 7 - 10, **dadurch gekennzeichnet, dass** sich der Reaktionsbehälter (10) zumindest unterhalb der Festbettauflage (14) in Richtung eines Auslasses (16) verjüngt.

12. Bioreaktor nach einem oder mehreren der Ansprüche 7 - 11, **gekennzeichnet durch** mindestens ein unterhalb der Festbettauflage (14) angeordnetes Filterelement (64, 66).

13. Bioreaktor nach Anspruch 12, **dadurch gekennzeichnet, dass**
(i) mehrere Filterelemente (64, 66) vorgesehen sind, deren Maschenweite/Porengröße in Richtung des Auslasses (16) abnimmt; und/oder
(ii) eine dem Filterelement (64, 66) zugeordnete Spüleinrichtung (68, 70) zur Reinigung des Filterelements (64, 66) vorgesehen ist; und/oder
(iii) das Filterelement (64, 66) in dem Reaktionsbehälter (10) geneigt angeordnet ist.

14. Bioreaktor nach einem oder mehreren der Ansprüche 8 - 13, **dadurch gekennzeichnet, dass** die Druckerzeugungseinrichtung (80) derart mit dem Reaktionsbehälter (10) verbunden ist, dass das Reaktionsmedium (56) zusätzlich durch das bzw. die Filterelemente (64, 66) hindurchpressbar ist.

## Claims

1. A cultivation process for microorganisms, comprising a combination of process steps characteristic of a trickle bed reactor with those characteristic of a fluidized bed reactor, wherein:
(a) microorganism cultures are grown on a solid bed under trickle bed reactor process operation, wherein the reaction medium is applied through trickle nozzles, the aeration is effected from below and/or within the solid bed, and the solution draining through the solid bed layer and containing the reaction/degradation products of the microorganism cultures is either recycled until the desired productivity, the desired product or the desired degradation product or the desired degree of degradation are achieved, or the drained solution is immediately treated further for further separation; and
(b) when a certain pressure loss through the packing of the solid bed is exceeded, the operation of the culture is temporarily switched from said trickle bed reactor process operation to a fluidized bed reactor process operation by introducing water or a nutrient solution and by introducing air.

2. The cultivation process according to claim 1, wherein:
(i) the microorganism cultures are selected from bacteria, yeasts, fungi, animal or plant cells and immobilized plant and animal tissues, and especially are filamentous fungi; and/or
(ii) in the fluidized bed operation, the medium is circulated by pumping to prevent overflowing of overgrown packing or overgrown solid bed components; and/or
(iii) the start-up of said microorganism culture is effected under trickle bed or fluidized bed reactor process operation; and/or
(iv) said reaction medium is selected from water, nutrient solution and solution with substrate to be reacted; and/or
(v) said reaction/degradation products of the microorganism cultures are proteins including enzymes and antibodies, secondary metabolic products, reacted substrates including alcohols, aldehydes, ketones, alkaloids, organic acids, pigments and flavoring agents, purified waste waters or converted xenobiotics and especially are proteins and secondary metabolites; and/or
(vi) said further separation comprises separating off solid particles and microorganisms by filtering or sedimenting and/or separating by means of ultrafiltration or chromatography; and/or
(vii) when a pressure loss through the packing of the fluidized bed of > 100 Pa per m of packing height or solid-bed filling, preferably of > 50 Pa/m, is exceeded, then the operation is switched from said trickle bed reactor process operation to said fluidized bed reactor process operation; and/or
(viii) the time for which said fluidized bed reactor process operation is effected is selected such that said exceeding of the pressure loss is reduced for the subsequent trickle bed reactor process operation, and preferably is at least 1 minute, especially from 10 min to 4 hours, and/or the desired production, material conversion or degradation effects are achieved by this additional operational mode.

3. The cultivation process according to claim 1 or 2, wherein:
(i) said process comprises the application of pressure, wherein the reaction medium is pressed through perforated trays and filter trays which may be present below the solid bed; and
(ii) said perforated trays and filter trays from (i) have different mesh numbers/pore sizes, especially decreasing, depending on the bed material, from 1 mm to the ultrafiltration range of about 5000 daltons, which allows for the successive isolation of particles of different orders of magnitude; and
(iii) a slant arrangement of said perforated trays and filter trays from (i) and (ii) enables the products to be eluted individually by washing with water/buffer and to be collected separately for further processing.

4. The cultivation process according to one or more of claims 1 to 3, wherein:
(i) all kinds of organic solid substrates including wood, annual plants, other plant material, humus, coal, all kinds of organic waste of both plant and animal origin as single materials or as mixtures are employed as said solid bed for the start-up of microorganism pure cultures or mixtures; and/or
(ii) packings, especially those consisting of plastic materials, ceramics, glass and mineral materials, active charcoal, silica or slags are employed as said solid bed, on which the microorganism cultures are induced to grow as pure cultures or mixtures free or immobilized; and/or
(iii) in the trickle bed reactor process operation, different organisms or organism mixed cultures are employed from those employed in the fluidized bed reactor process operation; and/or
(iv) enzymes, preferably hydrolases and/or oxidoreductases, are additionally added free or immobilized to the microorganisms of the solid bed; and/or
(v) during the culturing, co-enzymes are added which may be free or immobilized or co-immobilized with the respective enzyme; and/or
(vi) said co-enzymes of (v) are preferably selected from NAD, NADP, FAD, CoA, PAL, TPP and ATP.

5. The cultivation process according to one or more of claims 1 to 4, wherein:
(i) a separation, partial purification or concentration of the formed products or converted substrates is effected during the culturing; and/or
(ii) in the trickle bed reactor process operation, different organisms or organism mixed cultures are employed from those employed in the fluidized bed reactor process operation and, in particular, also different enzymes and co-enzymes are added in the different reactor operating modes; and/or
(iii) said process can be employed for the production of enzymes, for the production of secondary metabolites, for treating plant material, especially wood, annual plants, as a biocatalyst system in chemical synthesis, in the removal of noxious substances from waste water, and for the degradation of xenobiotics; and/or
(iv) in a successive production of enzymes, secondary metabolites, chemical intermediates and final products in the trickle bed reactor process operation and/or fluidized bed reactor process operation, the corresponding products are prepurified by the existing separation facilities of the bioreactor; and/or
(v) in the treatment of plant material, especially wood and annual plants, in the use in the removal of noxious substances from waste water and in the degradation of xenobiotics, the possibility of different process operation modes enables an improvement of the treatment performance or degradation performance.

6. The cultivation process according to claim 1, wherein:
(i) reactor control is effected by a feedback control by means of sensors present in the reactor for pH, pO₂ measurement, for measuring humidity, temperature and pressure; and/or
(ii) several bioreactors are arranged in series wherein the products of the first bioreactor are supplied to the second or the following bioreactors as substrates; and/or
(iii) the trickle bed culture segmented in the fluidized bed reactor process operation serves as a seed culture for the next scale-up step of the trickle bed and/or fluidized bed by passing it wholly or partly to the next scale-up step.

7. A bioreactor for the cultivation of microorganisms comprising:
a reaction container (10);
a solid-bed support (14) provided in said reaction container (10) for supporting a solid bed (12) on said solid-bed support (14);
trickle nozzles (18) provided above said solid-bed support (14) for trickling the solid bed (12) with a nutrient solution for the bioreactor to be operated in a trickle bed process;
an aerating means (30, 32, 34, 36) for aerating said solid bed (12) or the interior of the reactor (10) for the bioreactor to be operated in a fluidized bed process; and
a measured value monitoring means (58) for monitoring the pressure outside or within the solid bed (12).

8. The bioreactor according to claim 7, comprising:
a reaction container (10);
a solid-bed support (14) provided in said reaction container (10) for supporting a solid bed (12) on said solid-bed support (14);
trickle nozzles (18) provided above said solid-bed support (14) for trickling the solid bed (12) with a nutrient solution for the bioreactor to be operated in a trickle bed process; and
a pressurizing means (80) connected with said reaction container (10) for pressing reaction medium (56) through said solid-bed support (14).

9. The bioreactor according to claim 7 or 8, which is suitable for performing the process according to any of claims 1 to 6.

10. The bioreactor according to one or more of claims 7 to 9, **characterized by** a retention means (15) provided above said solid-bed support (14) for the fluidized bed reaction operation, and/or by a removal means (38) provided above said solid-bed support (14) and above said retention means (15) for medium discharge, especially in the flooded state.

11. The bioreactor according to one or more of claims 7 to 10, **characterized in that** said reaction container (10) is tapered towards an outlet (16) at least below said solid-bed support (14).

12. The bioreactor according to one or more of claims 7 to 11, **characterized by** at least one filter element (64, 66) provided below said solid-bed support (14).

13. The bioreactor according to claim 12, **characterized in that**:
(i) a plurality of filter elements (64, 66) are provided whose mesh numbers/pore sizes decrease towards said outlet (16); and/or
(ii) a flushing means (68, 70) assigned to said filter element (64, 66) is provided for purifying said filter element (64, 66); and/or
(iii) said filter element (64, 66) is arranged at an inclination in said reaction container (10).

14. The bioreactor according to one or more of claims 8 to 13, **characterized in that** said pressurizing means (80) is connected with said reaction container (10) in such a way that said reaction medium (56) can be additionally pressed through said filter element or elements (64, 66).

## Revendications

1. Procédé de culture de micro-organismes, comportant une combinaison d'étapes de procédé caractéristiques d'un réacteur à lit de ruissellement et d'étapes de procédé caractéristiques d'un réacteur à lit fluidisé, dans lequel
(a) des cultures de micro-organismes sont cultivées sur un lit fixe selon une conduite de procédé à réacteur à lit de ruissellement, le milieu de réaction étant fourni par des buses de ruissellement, la ventilation étant assurée en dessous et/ou à l'intérieur du lit fixe, et dans lequel la solution qui passe à travers la couche en lit fixe, solution qui contient des produits de réaction/décomposition des cultures de micro-organismes, soit est recyclée jusqu'à ce que la productivité désirée, le produit désiré ou le produit de décomposition désiré ou le degré de décomposition désiré soient atteints, soit la solution effluente est re-traitée directement pour séparation ultérieure, et
(b) en cas de dépassement d'une certaine perte de pression à travers le garnissage du lit fixe par introduction d'eau ou de bouillon de culture et par introduction d'air, la conduite de la culture passe, pour un certain laps de temps, de la conduite de procédé à réacteur à lit de ruissellement à une conduite de procédé à réacteur à lit fluidisé.

2. Procédé de culture selon la revendication 1, dans lequel
(i) les cultures de micro-organismes sont choisies parmi les bactéries, levures, champignons, cellules animales ou végétales et tissus végétaux et animaux immobilisés et sont, en particulier, des champignons filamenteux ; et/ou
(ii) dans le cas du régime en lit fluidisé, le milieu est pompé en boucle pour éviter un débordement de corps de remplissage développé ou de composants de lit fixe développés ; et/ou
(iii) l'enracinement de la culture de micro-organismes s'effectue au cours de la conduite en réacteur à lit de ruissellement ou en réacteur à lit fluidisé ; et/ou
(iv) le milieu de réaction choisi parmi l'eau, un bouillon de culture et une solution à substrat à convertir ; et/ou
(v) les produits de réaction/décomposition des cultures de micro-organismes sont des protéines y compris enzymes et anti-corps, des produits du métabolisme secondaire, des substrats convertis y compris alcools, aldéhydes, cétones, alcaloïdes, acides organiques, pigments et substances aromatiques, des effluents épurés ou des xénobiotiques convertis et sont, en particulier, des protéines et des métabolites secondaires ; et/ou
(vi) la séparation ultérieure comprend la séparation de particules solides et de microorganismes par filtration ou sédimentation et/ou séparation par ultrafiltration ou chromatographie ; et/ou
(vii) en cas de dépassement d'une perte de pression à travers le garnissage du lit fluidisé de > 100 Pa/m de hauteur de remplissage ou garnissage du lit fixe, de préférence de > 50 Pa/m, on passe de la conduite de procédé à réacteur à lit de ruissellement à la conduite de procédé à réacteur à lit fluidisé ; et/ou
(viii) la période pendant laquelle se déroule la conduite de procédé à réacteur à lit fluidisé est choisie de telle façon que, lors de la conduite de procédé à réacteur à lit de ruissellement subséquente, le dépassement de la perte de pression est repris, dure de préférence au moins 1 minute, en particulier 10 minutes à 4 heures, et/ou les effets de production, conversion de substances, décomposition attendus de ce régime additionnel, sont atteints.

3. Procédé de culture selon la revendication 1 ou 2, dans lequel
(i) le procédé comprend l'alimentation en pression, le milieu de réaction étant pressé à travers des fonds à tamis et fonds à filtre éventuellement existants sous le lit fixe ; et
(ii) les fonds à tamis et fonds à filtre de (i) présentent différentes largeurs de maille/grosseurs de pores, en particulier allant en décroissant, en fonction du matériau du lit, depuis 1 mm jusqu'à la plage d'ultrafiltration d'env. 5000 dalton, ce qui permet une isolation successive de particules de différents ordres de grandeur ; et
(iii) une disposition oblique des fonds à filtre et à tamis de (i) et (ii) permet d'éluer individuellement les produits par lavage à l'eau/tampon et de les collecter séparément pour traitement ultérieur.

4. Procédé de culture selon une ou plusieurs des revendications 1 à 3, dans lequel
(i) on met en oeuvre comme lit fixe tous les substrats solides organiques possibles, y compris bois, plantes annuelles, autre matériau végétal, humus, charbon, déchets organiques de toute nature d'origine tant végétale qu'animale sous forme de substances isolées ou de mélanges pour la production de cultures pures ou mélanges de microorganismes; et/ou
(ii) on met en oeuvre comme lit fixe des corps de remplissage, en particulier en matières plastiques, céramique, verre et matériaux minéraux, charbon actif, silice ou scories, sur lesquels on fait croître, sous forme libre ou immobilisée, les cultures de microorganismes en tant que cultures pures ou mélanges ; et/ou
(iii) dans la conduite de procédé à réacteur à lit de ruissellement, on met en oeuvre des organismes ou cultures mixtes d'organismes différents de ceux utilisés dans la conduite de procédé à réacteur à lit fluidisé ; et/ou
(iv) aux microorganismes du lit fixe on ajoute sous forme libre ou immobilisée, des enzymes supplémentaires, de préférence des hydrolases et/ou oxydoréductases ; et/ou
(v) lors de la culture on ajoute des co-enzymes qui peuvent être libres ou immobilisés ou co-immobilisés avec l'enzyme respectif ; et/ou
(vi) les co-enzymes de (v) sont choisis, de préférence, parmi NAD, NADP, FAD, CoA, PAL, TPP et ATP.

5. Procédé de culture selon une ou plusieurs des revendications 1 à 4, dans lequel
(i) lors de la culture s'opère une séparation, une purification partielle ou concentration des produits formés ou substrats convertis ; et/ou
(ii) au cours de la conduite de procédé à réacteur à lit de ruissellement, on met en oeuvre des organismes ou cultures mixtes d'organismes différents de ceux utilisés dans la conduite de procédé à réacteur à lit fluidisé et, en particulier, on ajoute aussi différents enzymes et co-enzymes aux différents modes de fonctionnement des réacteurs ; et/ou
(iii) le procédé de production d'enzymes, de production de métabolites secondaires, de traitement de matériau végétal, en particulier bois, plantes annuelles, est utilisable en tant que système biocatalyseur dans la synthèse chimique, dans l'élimination des substances nocives des effluents et pour la décomposition de la xénobiotique ; et/ou
(iv) lors d'une production successive d'enzymes, métabolites secondaires, produits chimiques intermédiaires ou finis dans la conduite de procédé à réacteur à lit de ruissellement et/ou dans la conduite de procédé à réacteur à lit fluidisé, les produits correspondants sont pré-épurés par les moyens de séparation existants du bioréacteur ; et/ou
(v) dans le traitement de matériau végétal, en particulier bois et plantes annuelles, en cas de mise en oeuvre dans l'élimination des substances nocives des effluents et lors de la décomposition de xénobiotique, la possibilité de conduire différents procédés permet une amélioration de la performance de traitement ou de la performance de décomposition.

6. Procédé de culture selon la revendication 1, dans lequel
(i) une commande du réacteur avec régulation de valeur de consigne/valeur réelle est réalisée par des capteurs existant dans le réacteur pour mesurer le pH, la pO₂, l'humidité, la température et la pression ; et/ou
(ii) plusieurs des bioréacteurs sont montés en série, dans lesquels les produits du premier bioréacteur sont introduits dans le second ou respectivement aux suivants sous forme de substrats ; et/ou
(iii) la culture à lit de ruissellement segmentée lors de la conduite de procédé du réacteur à lit fluidisé sert de culture d'inoculation pour l'étape suivante de montée en puissance du lit de ruissellement et/ou du lit fluidisé en étant transférée partiellement ou totalement dans l'étape de montée en puissance suivante.

7. Bioréacteur de culture de microorganismes comportant
un récipient de réaction (10),
un support de lit fixe (14) disposé dans le récipient de réaction (10) pour disposer un lit fixe (12) sur le support de lit fixe (14),
des buses de ruissellement (18) disposées au-dessus du support de lit fixe (14) pour l'arrosage du lit fixe (12) avec du bouillon de culture afin de permettre au bioréacteur de fonctionner selon le procédé à lit de ruissellement,
un dispositif de ventilation (30, 32, 34, 36) pour la ventilation du lit fixe (12) ou de l'espace intérieur du réacteur (10) afin de permettre au bioréacteur de fonctionner selon le procédé à lit fluidisé, et
une détection des valeurs de mesure (58) pour détecter la pression à l'extérieur du ou dans le lit fixe (12).

8. Bioréacteur selon la revendication 7 comportant
un récipient de réaction (10),
un support de lit fixe (14) disposé dans le récipient de réaction (10) pour disposer un lit fixe (12) sur le support de lit fixe (14),
des buses de ruissellement (18) disposées au-dessus du support de lit fixe (14) pour l'arrosage du lit fixe (12) avec du bouillon de culture afin dé permettre au bioréacteur de fonctionner selon le procédé à lit de ruissellement,
un dispositif (80) de génération de pression raccordé au récipient de réaction (10) pour presser le milieu de réaction (56) à travers le support de lit fixe (14).

9. Bioréacteur selon la revendication 7 ou 8, apte à mettre en oeuvre un procédé selon l'une des revendications 1 à 6.

10. Bioréacteur selon une ou plusieurs des revendications 7 à 9, **caractérisé par** un dispositif de retenue (15) disposé au-dessus du support de lit fixe (14) pour la conduite de réaction en lit fluidisé et/ou par un dispositif de prélèvement (38) disposé au-dessus du support de lit fixe (14) et au-dessus des éléments de retenue (15) pour l'évacuation du milieu, en particulier à l'état noyé.

11. Bioréacteur selon une ou plusieurs des revendications 7 à 10, **caractérisé en ce que** le récipient de réaction (10) s'effile au moins au-dessous du support de lit fixe (14) en direction d'une sortie (16).

12. Bioréacteur selon une ou plusieurs des revendications 7 à 11, **caractérisé par** au moins un élément filtrant (64, 66) disposé au-dessous du support de lit fixe (14).

13. Bioréacteur selon la revendication 12, **caractérisé en ce que**
(i) sont prévus plusieurs éléments filtrants (64, 66) dont la largeur de maille/grosseur de pores décroît en direction de la sortie (16) ; et/ou
(ii) un dispositif de rinçage (68, 70) associé à l'élément filtrant (64, 66) est prévu pour le nettoyage de l'élément filtrant (64, 66) ; et/ou
(iii) l'élément filtrant (64, 66) est disposé incliné dans le récipient de réaction (10).

14. Bioréacteur selon une ou plusieurs des revendications 8 à 13, **caractérisé en ce que** le dispositif (80) de génération de pression est raccordé au récipient de réaction (10) de façon telle que le milieu de réaction (56) peut, en outre, être pressé à travers le ou les élément(s) filtrant(s) (64, 66).
